(19) Europäisches Patentamt
European Patent Office
Office européen des brevets

(11) Publication number: **0 333 373 B1**

(12) **EUROPEAN PATENT SPECIFICATION**

(45) Date of publication of patent specification: **04.01.95**

(51) Int. Cl.[6]: **C07C 69/675**, C07C 67/14, C07C 69/732

(21) Application number: **89302291.3**

(22) Date of filing: **08.03.89**

(54) **Method for synthesizing acyloxycarboxylic acids.**

(30) Priority: **14.03.88 US 167544**

(43) Date of publication of application:
**20.09.89 Bulletin 89/38**

(45) Publication of the grant of the patent:
**04.01.95 Bulletin 95/01**

(84) Designated Contracting States:
**AT BE CH DE ES FR GB GR IT LI LU NL SE**

(56) References cited:
**AU-B- 423 814**

(73) Proprietor: **The Clorox Company**
**1221 Broadway**
**Oakland**
**California 94612 (US)**

(72) Inventor: **Rowland, Richard R.**
**106 Plaza Circle**
**Danville**
**CA 94526 (US)**

(74) Representative: **Smith, Sydney et al**
**Elkington and Fife,**
**Beacon House,**
**113 Kingsway**
**London WC2B 6PP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 333 373 B1

Rank Xerox (UK) Business Services
(3.10/3.09/3.3.3)

## Description

The present invention relates to acyloxycarboxylic acids prepared by an alcoholysis reaction, and particularly a method for synthesizing acyloxycarboxyiic acid in high yields. The synthesized acids are usefully converted to derivatives such as esters for bleaching applications.

## BACKGROUND OF THE INVENTION

Esters of acyloxycarboxylic acids have long been known for a wide variety of applications. Thus, for example, U.S. Patent 2,464,992, issued March 22, 1949, inventors Rehberg et al. teaches several methods for obtaining acyloxycarboxylic acid esters from starting materials such as glycolic or lactic acid with the esters useful as solvents, plasticizers, insecticides, insect repellents and chemical intermediates. An acyloxy acetic acid is disclosed as imparting excellent rust preventing characteristics to hydrocarbon mineral oils by U.S. Patent 2,659,697, issued November 17, 1953, inventor Wayo.

More recently, U.S. Patent 4,085,277, issued April 18, 1978, inventor Harada, discloses preparation of 2-cinnamoyloxyacetic acid as a starting compound in the preparation of a cephalosporanic acid derivative possessing antibacterial activity.

U.S. Patent Application Serial No. 928,070, (EP-A-0267047) entitled "Glycolate Ester Peracid Precursors" filed November 6, 1986, inventors Fong et al., of common assignment herewith, discloses compounds termed alkanoyloxyperacetic acid which are generated in situ when precursors are placed in aqueous solution with a source of hydrogen peroxide. These precursors are readily prepared from the acyloxycarboxylic acids synthesized by the present invention.

US Patent 2,503,699, issued April 11, 1950 , inventors Adelson et al., discloses the reaction of 1.9 equivalents acetyl chloride with 1.0 equivalent glycolic acid in a single reaction vessel to obtain acetylglycolic acid, which was isolated by evaporating excess acetyl chloride. However, the inventors report that the reaction was violent and evolved much hydrochloric acid. Acid chloride removal by evaporation to isolate product is not appropriate for longer chain acid chlorides. U.S. Patent 4,036,984 discloses adding various chlorides slowly under ice cooling into mixtures of various acids or alcohols with the mixtures including pyridine. However, when these known techniques are applied to the synthesis of acyloxycarboxylic acids such as, for example, octanoyloxyacetic acid, isolated yields of only about 40% to about 45% are obtained.

In AU-B-423,814 there is described a process for the production of acyl derivatives of hydroxy carboxylic acids, more particularly $C_{4-26}$ acyl derivatives of $\alpha$-hydroxy isobutyric acid. It is stated that the actual procedure for the preparation of the acyl derivatives comprises charging the reaction vessel with $\alpha$-hydroxy isobutyric acid and pyridine, and adding the acyl chloride dropwise to the mixture of the acid and pyridine. In another procedure which is said to be more economical it is stated that equimolar proportions of the isobutyric acid and the acyl chloride are reacted together with inert gas being bubbled through to drive off the hydrogen chloride generated.

## SUMMARY OF THE INVENTION

It is an object of the present invention to provide a method for synthesizing acyloxycarboxylic acids simply and in high yields, which acids are usefully converted to alkanoyloxyperacetic acids via ester precursors.

In one aspect of the present invention, a method for synthesizing an acyloxycarboxylic acid, useful as a starting material for conversion to various esters, comprises providing a reaction chamber, establishing sources of an $\alpha$-hydroxycarboxylic acid and an acid chloride, and repeatedly contacting substantially equimolar amounts of the $\alpha$-hydroxycarboxylic acid and the acid chloride within the reaction chamber. The sources of $\alpha$-hydroxycarboxylic acid and acid chloride are separated from one another, and are preferably repeatedly contacted at a predetermined, relatively slow rate within the reaction chamber. However, if the mixture within the reaction chamber is sufficiently agitated and cooled, then the $\alpha$-hydroxycarboxylic acid and acid chloride reactants can be contacted at relatively rapid rates.

The reaction chamber includes a basic component in an effective amount to neutralize a hydrogen chloride by-product during formation of the reaction product. The reaction product of this method is typically isolatable as at least 65% of theoretical yield and has the structure

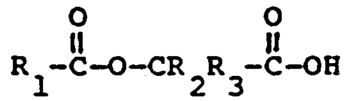

wherein $R_1$ is an alkyl group having two to twelve carbon atoms, for example two to ten carbon atoms, and $R_2$ is hydrogen, methyl, ethyl, or propyl and $R_3$ is hydrogen, methyl, ethyl, propyl, and substituted or unsubstituted phenyl or benzyl. The benzyl or phenyl group may be substituted with a methyl or ethyl group

In another aspect of the present invention, the just described reaction product, whether isolated or not, is converted to an ester which, when placed in aqueous solution with a source of hydrogen peroxide, results in a peracid having the structure (where $R_1$, $R_2$ and $R_3$ are as previously described):

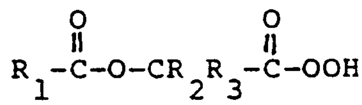

## DETAILED DESCRIPTION OF THE PREFERRED EMBODIMENTS

U.S. Patent Application Serial No. 928,070, (EP-A-0267047) entitled "Glycolate Ester Peracid Precursors", inventors Fong et al., filed November 6, 1986, and Serial No. 928,065, (EP-A-0267046) entitled "Acyloxynitrogen Peracid Precursors", inventor Zielske, filed November 6, 1986, both assigned to The Clorox Company, disclose ester conversions and applications useful with the present invention. The former application, for example, discloses preparations of acyloxycarboxylic acids which are converted to esters and utilized in bleach compositions to generate peracid when placed in aqueous solution with a source of hydrogen peroxide. However, the present inventive method provides acyloxycarboxylic acid preparations in considerably higher yields than disclosed.

Thus, Example I of EP-A-0267047 describes a synthesis of octanoyloxyacetic acid from glycolic acid and octanoyl chloride. The octanoyl chloride was added dropwise by means of an addition funnel to a flask charged with glycolic acid in chloroform and triethylamine with a minor amount of 4-dimethylaminopyridine. Isolated yield of the octanoyloxyacetic acid (with 90% purity) was 40% of theoretical yield. By contrast, and as described more fully hereinafter, isolated yield of octanolyoxyacetic acid (with greater than 90 wt. % purity) from practice of the present invention is at least about 65% of theoretical yield, and typically is greater than about 80 mole % crude yield.

Attempts to adapt the previously discussed Adelson et al. method from acetyl chloride (at 1.9 equivalents) with glycolic acid (at 1.0 equivalent) to a longer chain acid chloride, such as octanoyl chloride, result in an octanoyloxyacetic acid isolated yield of only 45%, by comparison to the above noted at least about 65% isolated yield in accordance with the present invention.

The disappointingly low isolated yields of desired acyloxycarboxylic acids by previously known methods are believed due, at least in part, to the bifunctional nature of alpha-hydroxycarboxylic acids since the acid chloride can react with either (or both) of the hydroxyl moieties. Practice of the inventive method is believed substantially to avoid the problems incurred with previous methods. That is, the present invention avoids the relatively low overall yields of reaction product, avoids the use of large excess of expensive starting material and avoids difficulty in isolation of the desired reaction product.

Practice of the inventive method typically provides the desired acyloxycarboxylic acid in about 80% to 85% crude yield, substantially avoids complicating side reactions such as generation of polyglycolic acid, and permits the desired reaction product to be readily isolated (if desired).

Practice of the inventive method synthesizes an acyloxycarboxylic acid, or reaction product, having the structure illustrated by Formula I below.

FORMULA I

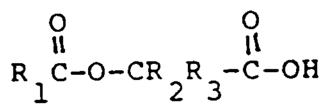

The $R_1$ substituent of the Formula I structure may be selected from alkyl groups (branched and unbranched) having two to twelve carbon atoms, that is from ethyl, propyl, butyl, pentyl, hexyl, heptyl, octyl, nonyl, decyl, undecyl and dodecyl groups. The $R_2$ substituent of the Formula I structure may be hydrogen or a lower alkyl group having one to three atoms. The $R_3$ substituent may be hydrogen, a methyl, ethyl or propyl group or a substituted or unsubstituted benzyl or phenyl group such as tolyl or xylyl.

This acyloxycarboxylic acid reaction product is formed by an alcoholysis reaction of an alpha-hydroxycarboxylic acid and an acid chloride in a reaction chamber. The total quantity of each reactant to be reacted in the reaction chamber is sometimes hereinafter referred to as an alpha-hydroxycarboxylic acid source and an acid chloride source. These reactant sources are a spaced distance from the reaction chamber and are separated from one another. This will be discussed more fully hereinafter.

Suitable alpha-hydroxycarboxylic acids for use as reactants in the inventive method include glycolic acid, lactic acid and the like, with some suitable such acids being illustrated by Table I.

Table I

| Acid Name | Structure |
| --- | --- |
| Glycolic | 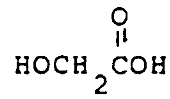 |
| Lactic | 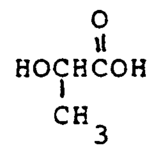 |
| 2-hydroxy butyric | 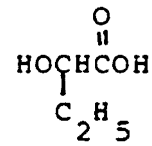 |
| α-hydroxy isobutyric | 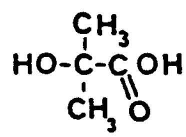 |
| Mandelic | 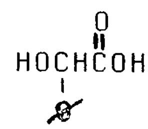 |
| β-phenyl lactic | 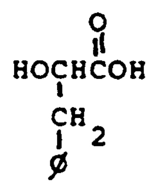 |
| 2-hydroxy-2-methyl-butyric | 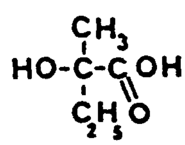 |
| α-hydroxy isocaproic | 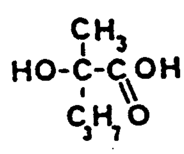 |

Glycolic acid is particularly preferred due to low cost, ready availability and suitable solubilities of the esters.

Suitable acid chlorides for use as reactants have the structure illustrated by Formula II below, where $R_1$ is an alkyl group (branched or unbranched) having 2 to 12 carbon atoms.

FORMULA II

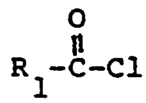

Preferred acid chlorides are hexanoyl chloride, octanoyl chloride, heptanoyl chloride, nonanoyl chloride and decanoyl, undecanoyl and dodecanoyl chlorides. Particularly preferred are octanoyl and nonanoyl chlorides.

It is extremely important that the alpha-hydroxycarboxylic acid and the acid chloride reactants be reacted by contacting substantially equimolar amounts within the reaction chamber. Excesses of one or the other components reduce selectivity of the desired reaction and thus reduce yield (illustrated as Reaction I below). For example, an excess of an acid reactant such as glycolic acid favors production of polyglycolates. An excess of the acid chloride reactant tends to lead to diketene formation.

The reaction is preferably conducted by contacting relatively small portions of the total reactant quantities in the reaction chamber. This may be achieved by pumping equimolar, metered quantities of both reactants (either neat or in solution) from the respective sources at a predetermined rate into the reaction chamber, and thus repeatedly contacting substantially equimolar amounts of the alpha-hydroxycarboxylic acid and the acid chloride.

This continuous and controlled reaction has been found substantially to prevent formation of a mixed anhydride as reaction product and thus to retard formation of undesired esters. For convenience, the reaction in which the acyloxycarboxylic acid is formed is illustrated as Reaction I.

REACTION I

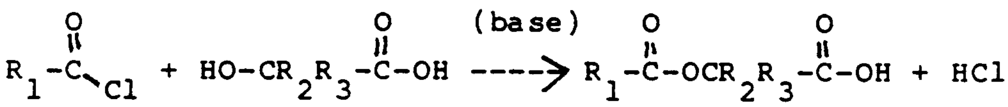


The contents of the reaction chamber are preferably agitated during the repeated contact of reactants, and a predetermined rate of pumped, metered quantities of reactants may be from about 0.1 to about 10.0 mole per liter per hour, more preferably about one mole per liter per hour for each reactant. However, as will be understood, the particular predetermined rate will be dependent upon the reaction vessel geometry, the mixing efficiency and the heat exchanging capacity of the system being used. Means known to the art, such as metering pumps for each reactant source, are a convenient way to pump such controlled quantities at the predetermined rate. The reaction is exothermic, and thus the reaction chamber is preferably cooled during the reaction by means well known to the art.

Where the alpha-hydroxycarboxylic acid is a solid, then a solvent is desirable to solubilize the alpha-hydroxycarboxylic acid within the reaction chamber during the repeated contact with the acid chloride. Suitable solvents are aprotic, polar and vary from water immiscible to water miscible, and preferably do not generate much heat from solvation. Preferred solvents are acetone, dichloromethane, acetonitrile, methyl ethyl ketone, diethyl ether, tetrahydrofuran, glyme, dioxane and ethyl acetate, most preferably acetone. The solvent is preferably present within the alpha-hydroxycarboxylic acid source, so that solubilized quantities can be conveniently flowed to the reaction chamber. Solvents may also be present within the reaction chamber to facilitate agitation and cooling of the reaction mixture.

During the reaction, the reaction chamber includes a basic component in an effective amount to neutralize the hydrogen chloride by-product that forms as the reaction proceeds. Suitable bases are believed to perform a dual function in not only neutralizing the hydrogen chloride by-product, but in apparently also acting to promote or catalyze the reaction. Preferred bases are tertiary amines such as pyridine, dimethylaminopyridine, triethylamine, t-propylamine, N-methylpiperidine and polymeric tertiary amines or cross-linked resins, such as polyvinylpyridine divinylbenzene, BIO-REX5 intermediate base anion exchange resin, AG4-X4 or AG3-X4A weakly basic anion exchange resin (the latter three being available from BIO-RAD Laboratories). The base preferably is used in about a stoichiometric amount (with respect to the reactants illustrated in Reaction I), and must not react with the acid chloride reactant.

The acyloxycarboxylic acid reaction product is contemplated for use as a bleach precursor having the general structure illustrated by Formula III.

FORMULA III

$$R_1-\overset{O}{\overset{\|}{C}}-OCR_2R_3-\overset{O}{\overset{\|}{C}}-L$$

where $R_1$, $R_2$ and $R_3$ are as previously described, and L is a leaving group. The carbonyl carbon of Formula III adjacent the leaving group is preferably esterified, and will have the leaving group bonded through the ester linkage.

Suitable leaving groups include derivatives of substituted or unsubstituted phenols, oximes, N-hydroxyimides, and amine oxides. These various suitable leaving groups are more fully described in previously noted EP-A-267046 and EP-A-267047.

The conversion of reaction product, prepared in accordance with the invention, to an ester is preferably via an acid chloride of the reaction product, as will be exemplified hereinafter. Such an ester, or bleach precursor, is usefully formulated with a solid source of peroxide, such as an alkaline peroxide, in amount effective to perhydrolyze the precursor and thus to provide effective bleaching. Suitable such sources of peroxide include sodium perborate monohydrate, sodium perborate tetrahydrate, sodium carbonate peroxyhydrate, sodium pyrophosphate peroxyhydrate, urea peroxyhydrate, sodium peroxide, and mixtures thereof. Sodium perborate monohydrate and sodium perborate tetrahydrate are particularly preferred alkaline peroxides for combination with such precursors as a dry bleach composition or, when surfactant is included, as a dry laundering and bleaching composition.

The source of peroxide (that is, compounds yielding hydrogen peroxide in an aqueous solution) itself constitutes a peroxygen bleaching compound. However, bleach compositions including peroxyacid precursor and peroxide source together provide better bleaching, particularly at temperatures below about 60 °C, than the peroxide source alone.

Two preferred bleach compositions including glycolate esters prepared in accordance with the inventive method are illustrated below.

| | |
|---|---|
| 15.6% | sodium perborate tetrahydrate |
| 19.0% | octanoyloxy acetic acid, p-phenyl sulfonate ester |
| 7.0% | nonionic surfactant |
| 15.0% | sodium carbonate |
| 43.4% | sodium sulfonate |
| 100.0% | |

| | |
|---|---|
| 15.5% | sodium perborate tetrahydrate |
| 16.8% | octanoyloxy acetic acid, t-butyl phenol ester |
| 7.0% | nonionic surfactant |
| 15.0% | sodium carbonate |
| 45.7% | sodium sulfate |
| 100.0% | |

Of course, a variety of other laundry adjuvants such as brighteners, fragrances, stabilizers, colorants, and the like, may be incorporated into such compositions.

Aspects of the invention will now be exemplified.

Example I

Synthesis of Octanoyloxy Acetic Acid (OOAA)

15.8 gm (0.20 mole) pyridine and 50 ml. acetone were combined in a 250 ml. round bottomed-three necked flask equipped with magnetic stir bar and two addition funnels, and cooled in an ice water bath with stirring. One addition funnel was charged with 15.5 gm (0.20 mole) glycolic acid dissolved in approximately 75 ml. acetone. The other addition funnel was charged with 32.5 gm (0.20 mole) octanoyl chloride. The contents of each addition funnel were added simultaneously to the cooled, stirred pyridine solution so that the complete addition of each component was continuous over one hour. The resultant slurry (a precipitation of pyridine hydrochloride was noted) was stirred an additional 45 minutes at ice bath temperature, and then at room temperature for another 30 minutes: Solvent was then removed by rotary evaporation at reduced pressure and 45° C. The residual oil was dissolved in 200 ml dichloromethane and this was extracted with 3 x 150 ml. of 4% aqueous HCl. The dichloromethane layer was dried over sodium sulfate, decanted and the solvent removed by rotary evaporation. Drying on high vacuum for 4 hours left OOAA and 8.0% octanoic acid by weight, giving a crude yield for OOAA of 84%(mole). Recrystallization from 200 ml. hexane gave 27 g of a white crystalline product melting at 50 to 52°C, which was determined to be 99% wt. OOAA, giving a 67% (mole) yield of pure octanoxyloxyacetic acid.

Example II

Synthesis of Octanoxyloxy Acetyl Chloride

101.1 gm (0.5 mole) octanoyloxy acetic acid and 83 gm (0.65 mole) oxalyl chloride are combined in a 1 liter round bottom flask with a magnetic stir bar and a $CaSO_4$ drying tube (note: a little hexane or petroleum ether can be added if the solid does not completely dissolve). The reaction is stirred at room temperature while rapid gas evolution is noted, then gradually heated to 40-50°C and held there for 2 hours, then at 65-70°C for one hour (note: the reaction can also be run at room temperature overnight with the advantage that it remains colorless). The slightly yellow solution is then heated to 60-70°C under aspirator pressure for one to 1-1/2 hours to remove excess oxalyl chloride. After cooling to room temperature the oil is diluted with 400 ml petroleum ether (bp 30-60°C) and extracted with 3x200 ml ice water (caution: gas evolution can be vigorous!). The organic layer is dried over $MgSO_4$, filtered and roto vapped to a clear straw colored oil, weight = 115.7 gm (110.4 gm theoretical). IR shows no acid-OH stretch and two carbonyls at about 1812 $cm^{-1}$ and at about 1755 $cm^{-1}$.

Example III

Synthesis of Octanoyloxy Acetic Acid, Phenyl Sulfonate Ester

17.3 gm (.079 mole) octanoyloxy acetyl chloride and 17.0 gm (.087 mole) sodium-p-phenol-sulfonate (dried at 120°C in vacuo for 16 hours) were combined in a 250 ml round bottom flask with a magnetic stir bar. 30 ml of ethylene glycol-dimethyl ether (glyme) was added, and the slurry stirred with cooling in an ice-water bath. 7.8 gm (.077 mole) triethylamine was placed in an additional funnel equipped with a $CaSO_4$ drying tube and this was added dropwise to the above slurry over 1/2 hour. The reaction becomes very thick and more glyme (or ethyl ether) can be added at this time to enable good stirring. The reaction was stirred for two hours at room temperature, diluted with ethyl ether and stirred one hour more. The reaction was filtered on a coarse glass frit, washed with several portions of ethyl ether, sucked dry for one hour and dried in vacuo at room temperature. Weight of product: 39 gm (theoretical wt. = 42.1 gm).

This material can be recrystallized from 60/40 (vol/vol) IpA/water in an approximate 3 to 4:1 (wt./wt.) ratio of solvent to ester reaction mixture to give an approximate 40-60% yield of ester ($90^{+}$% in purity).

Example IV

Synthesis of Hexanoyloxy Acetic Acid

10.1 gm (0.10 mole) triethylamine (TEA), 10 drops of pyridine, and 15 ml acetone were combined in a 250 ml round bottomed, three-necked flask equipped with magnetic stir bar and two addition funnels, and cooled in an ice water bath with stirring. One addition funnel was charged with 7.61 gm (0.10 mole) glycolic acid dissolved in 30 ml acetone. The other funnel was charged with 13.5 gm (0.10 mole) hexanoyl chloride. The contents of each addition funnel were added simultaneously to the cooled, stirred TEA/pyridine solution so that the complete addition of each component was continuous over 20 minutes (note: a heavy white precipitate, presumably TEA hydrochloride, formed during the addition). The reaction was stirred an additional one hour, filtered and the isolated salts washed with acetone, which was combined with the initial filtrate. Solvent was removed by rotary evaporation leaving a sweet smelling oil, which was dissolved in 150 ml di-ethyl ether, and extracted with 2x200 ml of 1% aqueous HCl. The ether layer was dried over magnesium sulfate, filtered and rotary evaporated to an oil weighing 20 gm of 74% wt. purity by GC, for a yield of 85% mole. No hexanoic or glycolic acids were found in the product, which was used without further purification.

Example V

Synthesis of Hexanoyloxy Acetyl Chloride

8.7 gm (0.05 mole) of hexanoyloxyacetic acid and 12.7 gm (0.10 mole) of oxalyl chloride were mixed together at room temperature. The reaction was heated gradually over one hour at 50-60 °C for about two hours. Excess oxalyl chloride was removed under reduced pressure to yield an oil that exhibits no -OH stretch by IR. Weight was 9.6 gm.

Example VI

Synthesis of Sodium, n-Hexanoyloxyacetate, p-phenylsulfonate

9.2 gm (0.04 mole) of n-hexanoyloxyacetyl chloride was added dropwise to an ice-cooled slurry of 9.0 gm (0.046 mole) sodium, p-phenolsulfonate (dried four hours at 110 °C in vacuo) and 5.5 gm (0.045 mole) triethylamine in 45 ml diglyme in a 100 ml round bottom flask fitted with a stirrer and low temperature thermometer. The reaction mixture was stirred for two hours at 0-4 °C, diluted with 100 ml ethyl ether, and filtered. The white solid precipitate was triturated with 3x100 ml of warm isopropanol and the solid was vacuum filtered and dried overnight under vacuum.

Example VII

Synthesis of Octanyloxyacetate, t-butyl Phenol Ester

5.95 gm (.025 mole) octanoyloxyacetyl chloride dissolved in about 15 ml anhydrous ethyl ether was added dropwise to a solution containing 2-1/2 gm (.027 mole) pyridine and 4.70 gm (.031 mole) t-butyl phenol in about 100 ml pyridine over one-half hour, with the solution being maintained at a temperature of 0-4 °C in an ice bath and stirred via a magnetic stir bar. The reaction was stirred at 5-10 °C for about 2 hours, filtered and then diluted to about 200 ml with ethyl ether. This was washed with 3 times 100 ml of 4% hydrochloric acid, 1 times 150 ml water, 2 times 100 ml of 10% sodium carbonate solution, then dried over sodium sulfate. The product was filtered and roto-vapped to yield a yellow oil, which was chromatographed on 60 gm of silica gel with 4% ethyl ether/petroleum ether distillate, yielding 5.3 gm of ester product determined to be 99.9 wt. % in purity by GC, saponification and NMR-13C.

Example VIII

Synthesis of Mixed Octanoyloxy/Decanoyloxy Acetic Acids

Reaction 1: 156.5 gm (1.01 equivalents) of $C_8$ /$C_{10}$ mixed aliphatic acids (63 mole % $C_8$ and 37 mole % $C_{10}$ ) and 114 ml (approx. 165 gm, 1.3 mole) oxalyl chloride were combined in a 1000 ml round

bottomed flask equipped with a magnetic stir bar and a calcium sulfate drying tube. The resultant solution was stirred for 19 hours (note: vigorous gas evolution ensued upon mixing of the two reactants). Excess oxalyl chloride was removed by warming of the reaction under reduced pressure for one hour. The residue was taken up in 300 ml of hexane and this solution was extracted with 5x250 ml of ice cold water. The hexane layer was dried over sodium sulfate, filtered and the solvent removed by rotary evaporation, leaving 184.5 gm of light straw colored oil. (No -OH by IR, and a strong $V_{C=O}$ at 1803 cm).

Reaction 2: A two liter, three-necked round bottom flask, equipped with mechanical stirrer and two addition funnels, was flame dried and charged with 79.5 gm (1.0 mole) pyridine in 200 ml acetone, which was then chilled in an ice water bath. One addition funnel was charged with the product from reaction 1 above, and the other charged with 84 gm (1.1 mole) glycolic acid in 300 ml acetone. The contents of the two addition funnels were added simultaneously and continuously to the stirred, chilled pyridine solution over 50 minutes. The reaction was then stirred an additional 2-1/2 hours at ice bath temperature. Solvent was removed by rotary evaporation and the oily residue dissolved in 50 ml dichloromethane, which solution was then extracted with 5x350 ml 5% aqueous HCl and 1x600 ml saturated NaCl, dried over sodium sulfate, filtered and rotary evaporated to a thick oil which soldified upon standing. Pumping off the residual solvent under high vacuum left 199 gm of solid which was determined to be 54.7% mole octanoyloxy acetic acid and 34.1% mole decanoyloxy acetic acid by GC. Overall yield of the two step reaction was 83.4% mole for the combined acyloxy acetic acids.

Example IX

Synthesis of Mixed Octanoyl/Decanoyl-Oxyacetic Acids, Sodium Phenol Sulfonate Esters

198 g of mixed octanoyl/decanoyl oxyacetic acids from Example VIII were melted on a warm (50 °C) oil bath in a 1000 ml round bottom flask. 113 ml of oxalyl chloride were added to the liquified acids and the reaction stirred by magnetic stir bar overnight at room temperature. The reaction was then warmed to 50 °C on an oil bath and excess oxalyl chloride removed at water aspirator pressure for 3-1/2 hours. The oily residue was dissolved in 700 ml hexane and washed with 3 x 250 ml of ice cold water. The hexane layer was dried over $Na_2SO_4$, filtered, and rotary evaporated to a light yellow oil weighing 219 gm. IR of this material exhibited no -OH stretch and C=O stretch at 1760 $cm^{-1}$ and 1820 $cm^{-1}$. These acid chlorides were then esterified as follows:

219 gm of the acid chlorides, 220 gm of sodium phenol sulfonate (from di-hydrate dried in vacuo at 120 °C for 48 hours), and 800 ml of anhydrous glyme were combined in a flame dried 2-1/3 necked Morton flask, equipped with a mechanical stirrer and addition funnel, and placed in an ice-water bath. The addition funnel was charged with 120 gm of triethylamine, which was added dropwise over one hour to the rapidly stirred, cooled acid chloride/phenol slurry. Over the time of addition of the amine the reaction mixture became so thick that an additional 500 ml of glyme was added to enable efficient stirring to proceed. Upon completion of the amine addition the reaction was stirred 1/2 hour longer, by which time it had become unstirrable. The reaction was allowed to stand two hours, and then filtered on a C-frit Buchner funnel. The filter cake was washed with 1000 ml of ethyl ether and sucked dry overnight. The residue was dried in vacuo leaving 470 gm of off-white powder, which contained 62% wt. of the desired esters, corresponding to a 74% mole conversion based on the starting ($C_8$/$C_{10}$) acid mixture. After two recrystallizations from IPA/water (approximately 1200 to 1800 ml each) 194 gm. of 95.7% wt. of the desired esters was obtained. Overall yield from the starting acids was 54% mold for the four reaction steps (Examples VIII and IX). The product contained less than 1.0% each of the $C_8$/$C_{10}$ acyl-oxy benzene sulfonate esters (HPLC).

Example X

Synthesis of 2-Hexanoyloxy-2-Methyl-Butyric Acid

11.8 gm (0.10 mole) 2-methyl-2-hydroxy-butyric acid was dissolved in 50 ml acetone and placed in one of two 125 ml addition funnels attached to a 250 ml three-necked round bottom flask charged with 8.0 gm (0.10 mole) pyridine in 50 ml acetone cooled in an ice-water bath. The other addition funnel was charged with 13.46 gm (0.10 mole) hexanoyl chloride. The contents of the two addition funnels were simultaneously added dropwise over 20 minutes to the stirred/cooled pyridine solution. The reaction was then stirred for two hours at 4-15 °C, then the solvent was removed by rotary evaporation. The oily residue was dissolved in 200 ml dichloromethane and this solution extracted with 5x150 ml 3% aqueous HCl, then washed with 1x200 ml deionized water. The organic layer was dried over $Na_2SO_4$ , filtered and solvent removed by

rotary evaporation, leaving 20.4 gm of light yellow oil (IR shows $V_{C=O}$ at 1745 and 1728 $cm^{-1}$). $^{13}C$ NMR saponifi-cation and GC analysis shows this material to be 99% the desired product, for an overall yield of 93%.

Example XI

Synthesis of 2-Octanoyl-Mandelic Acid

15.2 gm (0.10 mole) dl-mandelic acid was dissolved in 50 ml acetone and placed in one of two 125 ml additional funnels attached to a 250 ml three-necked round bottom flask charged with 8.0 gm (0.10 mole) pyridine in 50 ml acetone cooled in an ice water bath. The other addition funnel was charged with 16.3 gm (0.10 mole) octanoyl chloride. The contents of the two addition funnels were simultaneously added dropwise over 20 minutes to the stirred/cooled pyridine solution. The reaction was removed by rotary evaporation. The residue was dissolved in 200 ml diethyl ether and this solution was extracted with 5x150 ml 5% HCl, and then washed with 1x200 ml deionized water. The organic layer was dried over $Na_2SO_4$ , filtered and the solvent removed by rotary evaporation. After drying in vacuo there remained 28.2 gm of oil. GC and $^{13}C$ NMR determined this material to be 83.1% of the desired product, for an overall 84.2% yield.

Example XII

Synthesis of 2-Hexanoyl Mandelic Acid

15.2 gm (0.10 mole) dl-mandelic acid was dissolved in 50 ml acetone and placed in one of two 125 ml additional funnels attached to a 250 ml three-necked round bottom flask charged with 8.0 gm (0.10 mole) pyridine in 50 ml acetone cooled in an ice water bath. The other addition funnel was charged with 13.46 gm (0.10 mole) hexanoyl chloride. The contents of the two addition funnels were simultaneously added dropwise to the stirred, cooled pyridine solution. The reaction was then stirred at room temperature for two hours, at which time the solvent was removed by rotary evaporation. The oily residue was dissolved in 200 ml $CH_2Cl_2$ and this solution was extracted with 4x150 ml 3% aqueous HCl, and washed with 1x200 ml deionized water. The organic layer was dried over $Na_2SO_4$, filtered and the solvent removed by rotary evaporation, and dried in vacuo leaving 24.9 gm of oil. $^{13}C$ NMR and GC analysis determined this material to be 89.5% desired product, for an overall 89% yield.

Preparation of an oxime derivative is preferably by obtaining an acid chloride (illustrated by Example II and Example V), reacting with acetone oxime in a solvent such as TSF dropwise with rapid stirring, in a manner analogous to Example I of previously noted application Serial No. 928,065. The acyloxycarboxylic acids just exemplified by Examples I, IV, VIII, X, XI and XII are summarized by Table II.

TABLE II

| Example | Acid Prepared | Crude Yield (mole%) |
|---|---|---|
| I | 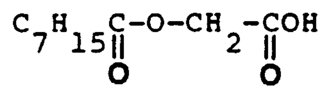 | 84 |
| IV | 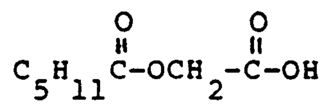 | 85 |
| VIII | 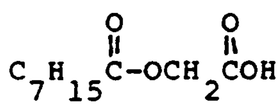 | 83.4 |
| | --and-- | |
| | 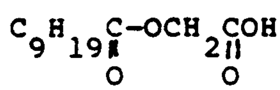 | |
| X | 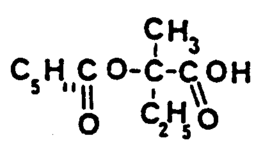 | 93.0 |
| XI | 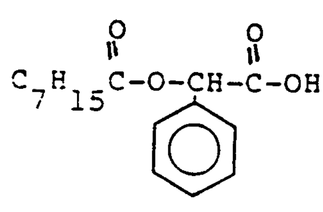 | 84.3 |
| XII | 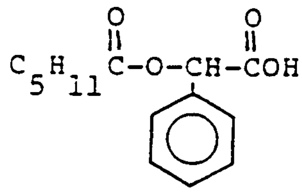 | 89.0 |

As may be seen from Examples I, IV, VIII, X, XI and XII, yield of crude acid products prepared by the inventive method ranged from 83.4% to 93%. While these acyloxycarboxylic acids may then be isolated before conversion to an ester for use as a bleach precursor, such isolation is often not necessary. Example

II illustrates use of an isolated acyloxycarboxylic acid (prepared as in Example I) to the chloride derivative and Example III illustrates conversion of such chloride to the p-phenyl sulfonate ester; however, Examples VIII and IX illustrate preparation of ester derivatives without an isolation of acyloxycarboxylic acid. Thus, the present invention provides a method for synthesizing acyloxycarboxylic acids simply and in high yields, which acids are usefully converted (with or without isolation) to ester precursors of alkanoyloxy peracetic acids.

## Claims

**1.** A method for synthesizing an acyloxycarboxylic acid comprising:

providing a reaction chamber, the reaction chamber including a basic component;

establishing sources of an α-hydroxy carboxylic acid and an acid chloride, both sources being separated from the reaction chamber and separated from one another; and,

repeatedly and simultaneously contacting substantially equimolar amounts of the α-hydroxy carboxylic acid and the acid chloride within the reaction chamber from the sources thereof to form an isolatable reaction product within the reaction chamber, the reaction product having the structure

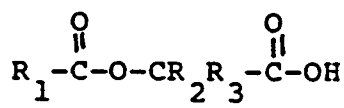

(I)

wherein $R_1$ is an alkyl group having two to twelve carbon atoms, $R_2$ is hydrogen, a methyl, ethyl or propyl group, and $R_3$ is hydrogen, a methyl, ethyl, or propyl group or a substituted or unsubstituted benzyl or phenyl group, the basic component within the reaction chamber being present in an effective amount to neutralize HCl by-product during formation of the reaction product.

**2.** The method as in claim 1 wherein the basic component is a tertiary amine.

**3.** The method as in claim 1 or claim 2 wherein the basic component is selected from pyridine, dimethylaminopyridine, triethylamine, t-propylamine, N-methylpiperidine and a polymeric tertiary amine.

**4.** The method as in any of claims 1-3 further comprising:

isolating the reaction product from the reaction chamber, the isolated reaction product being at least 65% of theoretical yield.

**5.** The method as in any of claims 1-4 wherein contents of the reaction chamber are agitated during the repeated contact of α-hydroxy carboxylic acid and acid chloride.

**6.** The method as in any of claims 1-5 wherein the α-hydroxy carboxylic acid is solubilized within the reaction chamber during the repeated contact with the acid chloride.

**7.** The method as in claim 6 wherein the α-hydroxy carboxylic acid is solubilized by an effective amount of an aprotic solvent.

**8.** The method as claimed in any of claims 1-7 wherein the α-hydroxy carboxylic acid is selected from glycolic acid, lactic acid, α-hydroxy propionic acid, α-hydroxy isobutyric acid, 2-hydroxy butyric acid, mandelic acid, β-phenyl lactic acid, 2-hydroxy-2-methyl butyric acid and α-hydroxy isocaptoic acid.

**9.** The method as in any of claims 1-8 wherein the acid chloride is selected from hexanoyl chloride, octanoyl chloride, decanoyl chloride, undecanoyl chloride, dodecanoyl chloride, nonanoyl chloride and heptanoyl chloride.

**10.** The method as claimed in any of claims 1-9 wherein the repeated contacting includes pumping metered quantities at a predetermined rate of the α-hydroxy carboxylic acid and the acid chloride from the sources thereof into the reaction chamber.

**11.** The method as claimed in claim 10 wherein the predetermined rate is approximately one M per hour.

**12.** A method for synthesizing a bleach precursor comprising:
preparing an acyloxycarboxylic acid of the formula (I) according to the method of claim 1; and converting the reaction product to an ester.

**13.** The method as in claim 12 wherein the ester conversion is via an acid chloride of the reaction product.

**14.** The method as in claim 12 wherein the reaction product ester has a structure

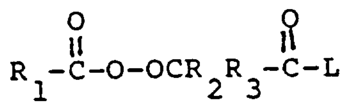

wherein $R_1$ $R_2$ and $R_3$ have the meanings given in claim 1, and L is a derivative of a substituted or unsubstituted phenol, an oxime, a N-hydroxyimide or an amine oxide.

**Patentansprüche**

**1.** Verfahren zur Synthese einer Acyloxycarbonsäure, dadurch **gekennzeichnet,** daß man
eine Reaktionskammer vorsieht, welche Reaktionskammer eine basische Komponente einschließt;
Quellen einer α-Hydroxycarbonsäure und eines Säurechlorids errichtet, wobei beide Quellen von der Reaktionskammer abgetrennt sind und voneinander abgetrennt sind; und daß man
wiederholt und gleichzeitig im wesentlichen äquimolare Mengen der α-Hydroxycarbonsäure und des Säurechlorids innerhalb der Reaktionskammer von ihren jeweiligen Quellen umsetzt, um ein isolierbares Reaktionsprodukt innerhalb der Reaktionskammer zu bilden, wobei das Reaktionsprodukt die Struktur:

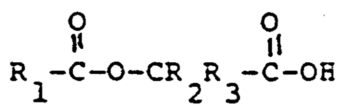 (I)

aufweist, worin $R_1$ eine Alkylgruppe mit 2 bis 12 Kohlenstoffatomen ist, $R_2$ ein Wasserstoffatom, eine Methyl-, Ethyl- oder Propylgruppe ist und $R_3$ ein Wasserstoffatom, eine Methyl-, Ethyl- oder Propylgruppe oder eine substituierte oder unsubstituierte Benzyl- oder Phenylgruppe ist,
und wobei die basische Komponente in der Reaktionskammer in einer wirksamen Menge vorhanden ist, um das HCl-Nebenprodukt während der Bildung des Reaktionsproduktes zu neutralisieren.

**2.** Verfahren nach Anspruch 1, dadurch **gekennzeichnet,** daß die basische Komponente ein tertiäres Amin ist.

**3.** Verfahren nach Anspruch 1 oder 2, dadurch **gekennzeichnet,** daß die basische Komponente aus Pyridin, Dimethylaminopyridin, Triethylamin, t-Propylamin, N-Methylpiperidin und einem polymeren tertiären Amin ausgewählt ist.

**4.** Verfahren nach einem der Ansprüche 1 bis 3, dadurch **gekennzeichnet,** daß man weiterhin das Reaktionsprodukt aus der Reaktionskammer isoliert, wobei die Ausbeute des isolierten Reaktionsproduktes mindestens 65% der Theorie beträgt.

**5.** Verfahren nach einem der Ansprüche 1 bis 4, dadurch **gekennzeichnet,** daß man den Inhalt der Reaktionskammer während des wiederholten Kontakts der α-Hydroxycarbonsäure mit dem Säurechlorid durchbewegt.

**6.** Verfahren nach einem der Ansprüche 1 bis 5, dadurch **gekennzeichnet,** daß man die α-Hydroxycarbonsäure in der Reaktionskammer während des wiederholten Kontakts mit dem Säurechlorid solubilisiert.

7. Verfahren nach Anspruch 6, dadurch **gekennzeichnet**, daß man die α-Hydroxycarbonsäure mit einer effektiven Menge eines aprotischen Lösungsmittels solubilisiert.

8. Verfahren nach einem der Ansprüche 1 bis 7, dadurch **gekennzeichnet,** daß die α-Hydroxycarbonsäure aus Glykolsäure, Milchsäure, α-Hydroxypropionsäure, α-Hydroxyisobuttersäure, 2-Hydroxybuttersäure, Mandelsäure, β-Phenylmilchsäure, 2-Hydroxy-2-methylbuttersäure und α-Hydroxyisocapronsäure ausgewählt ist.

9. Verfahren nach einem der Ansprüche 1 bis 8, dadurch **gekennzeichnet,** daß das Säurechlorid aus Hexanoylchlorid, Octanoylchlorid, Decanoylchlorid, Undecanoylchlorid, Dodecanoylchlorid, Nonanoylchlorid und Heptanoylchlorid ausgewählt ist.

10. Verfahren nach einem der Ansprüche 1 bis 9, dadurch **gekennzeichnet,** daß das wiederholte Kontaktieren das Einpumpen von abgemessenen Mengen der α-Hydroxycarbonsäure und des Säurechlorids mit vorbestimmter Geschwindigkeit von ihren Quellen in die Reaktionskammer einschließt.

11. Verfahren nach Anspruch 10, dadurch **gekennzeichnet,** daß die vorbestimmte Geschwindigkeit ungefähr 1 M pro Stunde ist.

12. Verfahren zur Synthese eines Bleichmittel-Vorläufers, dadurch **gekennzeichnet,** daß man
eine Acyloxycarbonsäure der Formel (I) nach dem Verfahren des Anspruchs 1 herstellt und daß man das Reaktionsprodukt in einen Ester umwandelt.

13. Verfahren nach Anspruch 12, dadurch **gekennzeichnet,** daß man die Esterumwandlung auf dem Wege über ein Säurechlorid des Reaktionsprodukts durchführt.

14. Verfahren nach Anspruch 12, dadurch **gekennzeichnet,** daß der als Reaktionsprodukt erhaltene Ester die Struktur:

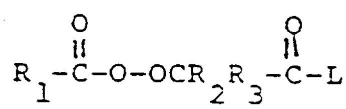

hat, worin $R_1$, $R_2$ und $R_3$ die in Anspruch 1 angegebenen Bedeutungen haben und L für ein Derivat eines substituierten oder unsubstituierten Phenols, eines Oxims, eines N-Hydroxyimids oder eines Aminoxids steht.

**Revendications**

1. Un procédé pour synthétiser un acide acyloxycarboxylique consistant à :
se pourvoir d'une chambre de réaction, la chambre de réaction comprenant un composant basique ;
établir des sources d'un acide α-hydroxycarboxylique et d'un chlorure d'acide, les deux sources étant séparées de la chambre de réaction et séparées l'une de l'autre ; et
mettre en contact, de façon répétée et simultanée, des quantités sensiblement équimolaires de l'acide α-hydroxycarboxylique et du chlorure d'acide dans la chambre de réaction à partir de leurs sources, pour former un produit réactionnel isolable dans la chambre de réaction, le produit réactionnel ayant la structure

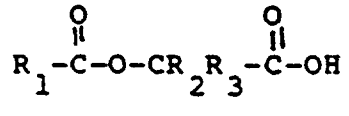
(I)

dans laquelle $R_1$ est un groupe alkyle ayant 2 à 12 atomes de carbone, $R_2$ est un hydrogène ou un groupe méthyle, éthyle ou propyle, et $R_3$ est un hydrogène ou un groupe méthyle, éthyle ou propyle, ou un groupe benzyle ou groupe phényle substitués ou non substitués, le composant basique dans la

chambre de réaction étant présent en une quantité efficace pour neutraliser l'HCl formé comme sous-produit lors de la formation du produit réactionnel.

2. Le procédé de la revendication 1, dans lequel le composant basique est une amine tertiaire.

3. Le procédé de la revendication 1 ou de la revendication 2, dans lequel le composant basique est choisi parmi la pyridine, la diméthylaminopyridine, la triéthylamine, la tert-propylamine, la N-méthylpipéridine et une amine tertiaire polymère.

4. Le procédé de l'une quelconque des revendications 1 à 3 comprenant de plus :
l'isolement du produit réactionnel de la chambre de réaction, le rendement du produit réactionnel isolé étant d'au moins 65 % de la théorie.

5. Le procédé de l'une quelconque des revendications 1 à 4, dans lequel le contenu de la chambre de réaction est agité lors du contact répété de l'acide $\alpha$-hydroxycarboxylique et du chlorure d'acide.

6. Le procédé de l'une quelconque des revendications 1 à 5, dans lequel l'acide $\alpha$-hydroxycarboxylique est solubilisé dans la chambre de réaction pendant le contact répété avec le chlorure d'acide.

7. Le procédé de la revendication 6, dans lequel l'acide $\alpha$-hydroxycarboxylique est solubilisé par une quantité efficace d'un solvant aprotique.

8. Le procédé de l'une quelconque des revendications 1 à 7, dans lequel l'acide $\alpha$-hydroxycarboxylique est choisi parmi l'acide glycolique, l'acide lactique, l'acide $\alpha$-hydroxypropionique, l'acide $\alpha$-hydroxyisobutyrique, l'acide 2-hydroxybutyrique, l'acide mandélique, l'acide $\beta$-phényllactique, l'acide 2-hydroxy-2-méthylbutyrique et l'acide $\alpha$-hydroxyisocaproïque.

9. Le procédé de l'une quelconque des revendications 1 à 8, dans lequel le chlorure d'acide est choisi parmi le chlorure d'hexanoyle, le chlorure d'octanoyle, le chlorure de décanoyle, le chlorure d'undécanoyle, le chlorure de dodécanoyle, le chlorure de nonanoyle et le chlorure d'heptanoyle.

10. Le procédé de l'une quelconque des revendications 1 à 9, dans lequel le contact répété comprend le pompage de quantités mesurées à un débit prédéterminé de l'acide $\alpha$-hydroxycarboxylique et du chlorure d'acide à partir de leurs sources dans la chambre de réaction.

11. Le procédé de la revendication 10, dans lequel le débit prédéterminé est d'environ une M par heure.

12. Un procédé de synthèse d'un précurseur d'agent de blanchiment comprenant :
la préparation d'un acide acyloxycarboxylique de formule (I) selon le procédé de la revendication 1 ; et
la conversion du produit réactionnel en un ester.

13. Le procédé de la revendication 12, dans lequel la conversion en ester est réalisée via un chlorure d'acide du produit réactionnel.

14. Le procédé de la revendication 12, dans lequel l'ester du produit réactionnel a pour structure

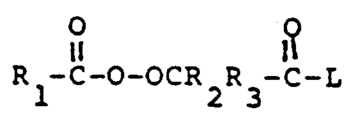

dans laquelle $R_1$, $R_2$ et $R_3$ ont les significations indiquées dans la revendication 1 et L est un dérivé d'un phénol, d'une oxime, d'un N-hydroxyimide ou d'un oxyde d'amine, substitués ou non substitués.